# EUROPÄISCHE PATENTSCHRIFT

(11) **EP 1 408 834 B1**
(45) Veröffentlichungstag und Bekanntmachung des Hinweises auf die Patenterteilung: **23.11.2005**
(21) Anmeldenummer: 02758065.3
(22) Anmeldetag: 01.07.2002
(51) Int. Cl.: A61B 5/103, A61F 13/02

(54) **MEDIZINISCHES INDIKATORPFLASTER**
MEDICAL INDICATOR PLASTER
EMPLATRE TEMOIN MEDICAL

(30) Priorität: 26.07.2001 DE 10136106; 26.07.2001 DE 10136107
(43) Veröffentlichungstag der Anmeldung: 21.04.2004
(73) Patentinhaber: Zick, Reinhard, 49803 Lingen/Ems (DE); Thissen, Rudolf, 41849 Wassenberg (DE); Cremerius, Alois, 40477 Düsseldorf (DE)
(72) Erfinder: Zick, Reinhard, 49803 Lingen/Ems (DE); Thissen, Rudolf, 41849 Wassenberg (DE); Cremerius, Alois, 40477 Düsseldorf (DE)
(74) Vertreter: von Creytz, Dietrich
(86) Internationale Anmeldenummer: PCT/DE2002/002373
(87) Internationale Veröffentlichungsnummer: WO 2003/011134

(56) Entgegenhaltungen:
- EP-A- 0 430 608
- WO-A-99/36017
- DE-A- 4 308 945
- DE-A- 19 617 327
- US-A- 5 511 561

## Beschreibung

Die Erfindung betrifft ein medizinisches Indikatorpflaster zum Applizieren auf die Fußsohle für die Anwendung bei der Früherkennung des diabetischen Fußes, wobei in den Körper des Pflasters mindestens ein durch das Gewicht des auf dem Pflaster stehenden Patienten zu aktivierender Druckindikator integriert ist. Das Indikatorpflaster kann auch als Meßfolie oder -pflaster bezeichnet bzw. ausgebildet sein.

Das Pflaster soll zur Anwendung in der Früherkennung des sogenannten diabetischen Fußes (kurz: DF) konzipiert werden. Dieser nimmt unter den verschiedenen Folgeerkrankungen der Diabetes mellitus (kurz: DM) eine Sonderstellung ein. Er verursacht immer noch Jahr für Jahr etwa 28.000 Amputationen in Deutschland und hat damit nicht nur einen Kostenfaktor ersten Ranges für die gesetzliche Krankenversicherung, sondern und vor allem für die betroffenen Patienten ein Übermaß an Schmerzen, Behinderungen und sozialen Nachteilen zur Folge.

In der Praxis geht es nur vordergründig um den Fuß, der nicht zum DF werden soll, darüber hinaus stellt nämlich der intakte Fuß ein Synonym für die erhaltene Mobilität des Diabetikers dar. Gerade diese ist aus folgendem Grund wichtig: Im Gesamtkonzept der DM-Behandlung hat bekanntlich die normnahe Blutzuckereinstellung (kurz: BZ-Einstellung) den höchsten Stellenwert. Diese normnahe BZ-Einstellung kann aber nur gelingen, wenn dem Patienten die Möglichkeit erhalten bleibt, seinen BZ-Spiegel durch regelmäßige, planmäßig betriebene Muskelaktivität positiv zu beeinflussen. Gerade dazu braucht er gesunde Füße.

Ein Diabetiker mit DF-Syndrom hat also nicht nur lokale Probleme mit seinen Füßen, sondern darüber hinaus auch ein Dauerproblem mit seinem BZ-Spiegel. Das Behandlungsziel einer normnahen BZ-Einstellung ist unter den Bedingungen des DF-Syndroms nur schwer, wenn überhaupt, zu erreichen. Damit drohen dem Patienten fast zwangsläufig weitere DM-Folgeschäden an Augen, Herz, Gefäßen, Nieren und im Magen-Darmtrakt. Die Früherkennung des DF oder, anders ausgedrückt, der Nachweis erhaltener Fußgesundheit ist von großem Interesse für Arzt und Patient.

In DE 100 18 790 A1 (nach dem Prioritätstag der vorliegenden Anmeldung veröffentlicht) wird ein Indikatorpflaster zur Diagnostik des DF-Syndroms beschrieben. Als Indikator enthält das Pflaster Kobalt-(II)-Chlorid, das in trockenem Zustand eine Blaufärbung und bei einem gewissen Feuchtigkeitsgehalt, welcher einer normalen Hautfeuchte entspricht, in Rotfärbung umschlägt. Wird ein solches Pflaster beispielsweise auf die Fußsohle aufgebracht, so kann nach kurzer Zeit festgestellt werden, ob ein Farbumschlag stattgefunden hat oder nicht. Im Bekannten wird definiert, daß ein fehlender Farbumschlag eine abnorme Hauttrockenheit anzeigt; diese kann einen Hinweis auf den diabetischen Fuß bilden.

In WO 97/31599 wird ein selbstklebender Indikator beschrieben, der es erlaubt, beispielsweise den Glukosegehalt des Blutes als chemische Reaktion anzuzeigen.

Der Indikator kann daher für eine chemische Nachweisreaktion des Diabetes mellitus eingesetzt werden.

Schließlich wird in EP 04 30 608 A1 ein Indikatorpflaster zur Diabetes-Diganose beschrieben, welches eine Klebschicht besitzt, die in Wasser lösliche Hydrokolloide enthält und welches als Indikator ein temperaturempfindliches Flüssigkristallband aufweist. Dieses Pflaster hat wie die anderen bekannten Pflaster einen relativ komplizierten technischen Aufbau und liefert seine Informationen als Folge thermisch-chemischer Reaktionen.

Der Erfindung liegt die Aufgabe zugrunde, im Wesentlichen mechanisch wirkende bzw. aktivierte Hilfsmittel bzw. Vorrichtungen zur DF-Früherkennung für möglichst alle bisher noch DF-symptomfreien Diabetiker zu schaffen. Diese Hilfsmittel sollen kostengünstig sowie aussagestark (d.h. wissenschaftlich validiert) und unkompliziert in der Anwendung sein.

Die erfindungsgemäße Lösung besteht für das Indikatorpflaster, in dessen Körper ein durch das Gewicht des auf dem Pflaster stehenden Patienten zu aktivierender - also druckempfindlicher - Druckindikator integriert ist, darin, daß der Druckindikator als druckfeste Kugel ausgebildet ist. Einige Verbesserungen und weitere Ausgestaltungen der Erfindung werden in den Unteransprüchen beschrieben.

Erfindungsgemäß bedeutet die Angabe, der Indikator sei durch das Gewicht des auf dem Pflaster stehenden Patienten zu aktivieren, daß die im Indikatorpflaster auf der Fußsohle befindliche Kugel von dem Patienten, je nachdem ob der Fuß gesund ist oder nicht als mechanische Schmerzquelle wahrgenommen oder - im Extremfall - überhaupt nicht bemerkt wird. Als Druckindikator kann zusätzlich eine handelsübliche Druck-Indikatorfolie im Pflaster vorgesehen werden. Eine solche Folie verändert bei einem bestimmten Druck irreversibel ihre Farbe. Die Folie kann im Rahmen der Erfindung so abgestimmt werden, daß sie eine Überschreitung des kritischen Druckes per Farbänderung anzeigt. Die für die Entstehung des diabetischen Fußes kritischen Drucke sind bekannt und in Normtabellen erfaßt.

Vorzugsweise soll die druckfeste Kugel an der der Fußsohle zuzuwendenden Unterseite des Pflasters angebracht werden. Bei Anwendung befindet sich dann die Kugel, vorzugsweise nicht sichtbar, zwischen Haut und Pflaster. Gegebenenfalls soll die Indikatorfolie auf der bei Anwendung von der Fußsohle abzuwendenden Oberseite des Pflasters vorgesehen werden, damit die Druckwirkung nicht durch Pflasterschichten gedämpft wird. Wenn das Pflaster Kugel und Druck-Indikatorfolie umfaßt, sollen erstere an der Unterseite und letztere an der Pflasteroberseite bevorzugt übereinander, so daß die Kugel Druck auf die Folie ausüben kann, angewendet werden.

Das erfindungsgemäß zu verwendende Pflaster kann vorzugsweise eine Klebefläche (an seiner Unterseite), mit deren Hilfe es an der Fußsohle zu fixieren ist, besitzen. Eine Klebefläche ist aber nicht an sich erfindungswesentlich; zum Befestigen des Pflasters können auch andere Mittel, z.B. ein Verband, benutzt werden. Der Begriff "Pflaster" wird also im Rahmen der Erfindung allgemein als Fixiermittel - gegebenenfalls Träger - des Druckindikators verstanden. Im Prinzip können Kugel und gegebenenfalls Indikatorfolie auch mit Hilfe daran selbst aufgebrachtem Klebstoff, an der jeweiligen Hautstelle fixiert werden. Es kann genügen, die Teile so zu befestigen, daß der Patient einige Schritte mit dem Pflaster gehen kann. Erfindungsgemäß kann eine handelsübliche Druck-Indikatorfolie in der Funktion eines Pflasters unmittelbar mit einer der druckfesten Kugeln ausgestattet und für das Fixieren an der jeweils zu untersuchenden Stelle vorbereitet werden.

Ferner, die erfindungsgemäß vorgesehene Kugel - grundsätzlich können auch zwei oder mehr Kugeln in ein Pflaster integriert werden - muß nicht notwendig die geometrische Kugelform besitzen. Im Rahmen der Erfindung können auch ovale oder unregelmäßige Formen, wie diejenige eines Steinchens, vorgesehen werden. Von einer Kugel wird - ähnlich wie bei dem Pflaster - nur zur Vereinfachung der Beschreibung gesprochen. Vorzugsweise kann ein handelsübliches Pflaster mit klebender Unterseite so präpariert werden, daß bei Anwendung die Kugel zwischen Haut- bzw. Wundauflage und dem Körper des Pflasters an einer vorgegebenen Hautstelle zu fixieren ist.

Gemäß weiterer Erfindung soll die in das Pflaster integrierte Kugel, vorzugsweise im Durchmesserbereich von größenordnungsmäßig 1,5 bis 5 mm, einen annährend maximalen, d.h. so großen Durchmesser haben, daß von dem Patienten ein unter seinen erkrankten Fuß (an der Trittfläche der Fußsohle) appliziertes Pflaster mit Kugel nicht mehr mit Sicherheit von einem Pflaster ohne eine solche Kugel zu unterscheiden ist; der gesunde Patient den Unterschied aber deutlich merkt. Eventuell kann die Kugelgröße individuell - z.B. durch Probieren - an die Empfindlichkeit und/oder das Körpergewicht des einzelnen Patienten angepaßt ausgewählt werden. Bei bisherigen Ausführungsbeispielen hat sich ein Kugeldurchmesser von etwa 2 bis 4 mm, d.h. in der Größenordnung von 3 mm, bewährt.

In Verbindung mit den Kugeln können handelsübliche Druck-Indikatorfolien mit den angegebenen Eigenschaften, z.B. mit getrennten oder, als einteilig gelieferte Doppelfolie, bereits verbundenen Folien, bestehend aus einer farbgebenden und einer sich als Antwort auf einen Mindestdruck färbenden Schicht, verwendet werden. Im Rahmen der Erfindung werden die Folie und/oder das Pflaster, gegebenenfalls die Kompressibilität des letzteren, so abgestimmt bzw. ausgewählt, daß die Folie eine Überschreitung eines kritischen Drucks sichtbar sichtbar erscheinen läßt.

Bei der Anwendung der erfindungsgemäßen druckfesten Kugel (gegebenenfalls zugleich mit Druck-Indikatorfolie) wird bevorzugt ein Pflaster-Set in einer Lieferform bestehend aus einem (mit der Kugel) präparierten und einem nicht präparierten Exemplar bereitgestellt. Dem Patienten wird nicht gesagt, welches der Pflaster präpariert ist (verblindete Untersuchung). Auf jeder Fußsohle des Patienten wird ein Pflaster fixiert. Man läßt den Patienten dann einige Schritte gehen und fragt ihn nach seinem Sinneseindruck, insbesondere soll der Patient mitteilen, ob er einen Unterschied bemerkt. Vorzugsweise sollen die beiden Pflaster an gleichen bzw. sich entsprechenden Stellen des rechten und linken Fußes, speziell an den eigentlichen Trittflächen der Fußsohle, befestigt werden. In manchen Fällen kann es günstig sein, die Untersuchung mit einem zweiten Pflaster-Set, aber mit umgekehrter Verteilung, zu wiederholen.

Der bei dem Patienten an dessen gesunden Fuß wegen der Kugel zu erwartende Sinneseindruck besteht in einem nachdrücklichen bis schmerzhaften Fremdkörpergefühl am Applikationsort des mit einer Kugel präparierten Pflasters; die Kugel wirkt dort im Wesentlichen ähnlich wie ein Stein im Schuh. Fehlt dieser Sinneseindruck oder wird er falsch lokalisiert, ist dies ein sicherer Hinweis auf ein DF-Frühstadium. - In diesem Sinne kann es vorteilhaft sein, die Kugel nahe der Pflasterunterseite, bevorzugt unmittelbar an der Unterseite des Pflasterkörpers, in das Pflaster zu integrieren. Dann nämlich wird die Wirkung der Kugel nicht durch Zwischenlagen - Schichten zwischen Haut und Kugel - gedämpft.

Im Rahmen der Erfindung wird von einer "druckfesten" Kugel wird gesprochen, weil die Druck-Indikatorfolien Farbkugeln enthalten können, die unter einem Mindestdruck zerquetscht werden, also gerade nicht druckfest sind. Da die druckfesten Kugeln gegebenenfalls Wirkungen auf die Haut haben, die Indikatorfolie aber selbst verändert werden soll, kann es günstig sein, die Kugel an der Pflasterunterseite und die Indikatorfolie an der Pflasteroberseite vorzusehen. Die Wirkung der Kugel wird dann nicht durch Zwischenschichten gedämpft, d.h. sie ist maximal, und eine Veränderung der außen liegenden Folie kann leicht erkannt werden.

Wenn die Druck-Indikatorfolie bei einen Pflaster-Set an beiden Pflastern und die Kugel an einem der Pflaster vorgesehen wird, können zugleich die protektive Sensibilität (z. B. die Oberflächen- und Tiefensensibilität sowie das Schmerzempfinden) und der kritische Druck an der Fußsohle geprüft werden.

Die Erfindung ermöglicht es erstmalig im Wesentlichen mechanisch in unkomplizierter und kostengünstiger Form, die entscheidenden Risikoparameter für den DF zu erfassen. Das erfindungsgemäße Indikatorpflaster ist wirtschaftlich vorteilhaft und wissenschaftlich fundiert, es verursacht niedrige Kosten und liefert eine beweiskräftige Aussage für einen gesunden oder kranken Fuß. Der Patient kann die Untersuchung - auch ohne den Arzt - selbst vornehmen.

Anhand der schematischen Darstellung eines Ausführungsbeispiels werden Einzelheiten der Erfindung erläutert. Es zeigen
- **Fig. 1**: einen Schnitt durch ein erfindungsgemäßes Indikatorpflaster; und
- **Fig. 2**: eine Draufsicht auf das Indikatorpflaster nach Fig. 1

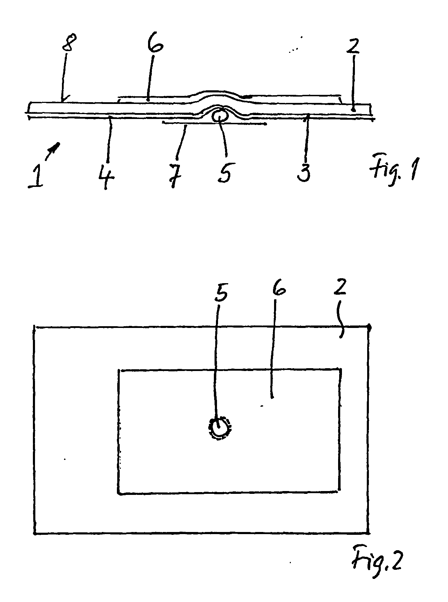

Das insgesamt mit 1 bezeichnete Indikatorpflaster nach Fig. 1 und 2 besteht im Wesentlichen aus einer Tragschicht 2 mit an deren Unterseite 3 vorgesehener Klebschicht 4. In das Indikatorpflaster 1 nach Fig. 1 sind eine druckfeste Kugel 5 und eine Druck-Indikatorfolie 6 integriert. Die Kugel 5 befindet sich nach Fig. 1 an der Unterseite der Tragschicht 2, vorzugsweise zugleich an der Unterseite der Klebschicht 4. Sie kann dort mit einer dünnen Folie 7 abgedeckt sein. Die Druck-Indikatorfolie 6 befindet sich nach Fig. 1 auf der der Unterseite 3 gegenüberliegenden Oberseite 8 der Tragschicht 2. Vorzugsweise sollen die Kugel 5 und die Druck-Indikatorfolie übereinander liegen, so daß die Kugel 5 bei Ausüben eines Drucks unmittelbar auf die Folie 6 wirkt.

## Patentansprüche

1. Medizinisches Indikatorpflaster (1) zum Applizieren auf die Fußsohle für die Anwendung bei der Früherkennung des diabetischen Fußes (DF), wobei in den Körper des Pflasters mindestens ein durch das Gewicht des auf dem Pflaster stehenden Patienten zu aktivierender Druckindikator integriert ist, **dadurch gekennzeichnet, daß** der Druckindikator als druckfeste Kugel (5) ausgebildet ist.

2. Indikatorpflaster nach Anspruch 1, **dadurch gekennzeichnet, daß** als Druckindikator zugleich mindestens eine druckfeste Kugel (5) und mindestens eine Druck-Indikatorfolie (6), bevorzugt übereinander, vorgesehen sind.

3. Indikatorpflaster nach Anspruch 1 oder 2, **dadurch gekennzeichnet, daß** die druckfeste Kugel (5) an der bei Anwendung der Fußsohle zuzuwendenden Unterseite (3) des Pflasters (1) angebracht ist.

4. Indikatorpflaster nach mindestens einem der Ansprüche 1 bis 3, **dadurch gekennzeichnet, daß** der Durchmesser der druckfesten Kugel, insbesondere in der Größenordnung von 1,5 bis 5 mm, vorzugsweise mit etwa 2 bis 4 mm, so ausgewählt ist, daß ein bei Anwendung unter der Fußsohle fixiertes Pflaster mit Kugel (5) von einem Patienten mit diabetischem Fuß nicht mit Sicherheit von einem Pflaster ohne Kugel zu unterscheiden ist.

5. Indikatorpflaster nach mindestens einem der Ansprüche 1 bis 4, **gekennzeichnet durch** eine Lieferform als Set bestehend aus einem Pflaster (1) mit Kugel (5) und einem Pflaster ohne Kugel.

6. Indikatorpflaster nach mindestens einem der Ansprüche 1 bis 5, **dadurch gekennzeichnet, daß** eine in das Pflaster integrierte Druck-Indikatorfolie (6) auf die bei Anwendung von der Fußsohle abzuwendende Oberseite (8) des Pflasters (1) aufgebracht ist.

## Claims

1. Medical indicator plaster (1) for application onto the sole of the foot for use in the early detection of a diabetic foot (DF), where at least one pressure indicator is integrated into the body of the plaster which is activated by the weight of the patient standing on the plaster, **characterised in that** the pressure indicator is in the form of a pressure-resistant ball (5).

2. Indicator plaster according to claim 1, **characterised in that** at least one pressure-resistant ball (5) and at least one pressure indicator film (6) are provided as the pressure indicator, preferably layered on top of one another.

3. Indicator plaster according to claim 1 or 2, **characterised in that** the pressure-resistant ball (5) is attached to the underside (3) of the plaster (1) which faces the sole of the foot when in use.

4. Indicator plaster according to at least one of claims 1 to 3, **characterised in that** the diameter of the pressure-resistant ball is selected to be in particular in the order of 1.5 to 5 mm, preferably about 2 to 4 mm, so that a plaster with a ball (5) attached to the sole of the foot when in use cannot be distinguished with certainty by a patient with a diabetic foot from a plaster without a ball.

5. Indicator plaster according to at least one of claims 1 to 4, **characterised by** the form of delivery in a set comprising a plaster (1) with a ball (5) and a plaster without a ball.

6. Indicator plaster according to at least one of claims 1 to 5, **characterised in that** a pressure indicator film (6) integrated into the plaster is applied onto the upper side (8) of the plaster (1) facing away from the sole of the foot when in use.

## Revendications

1. Emplâtre témoin médical (1) à appliquer sur la plante du pied pour une utilisation dans la détection précoce du pied diabétique (PD), dans lequel est incorporé -dans le corps de l'emplâtre- au moins un témoin de pression à activer par le biais du poids du patient qui se tient debout sur l'emplâtre, **caractérisé en ce que** le témoin de pression est réalisé sous la forme d'une bille (5) résistant à la pression.

2. Emplâtre témoin selon la revendication 1, **caractérisé en ce qu'**il est prévu comme témoin de pression au moins une bille (5) résistant à la pression et, à la fois, au moins une feuille témoin (6), les deux étant disposées, de préférence, l'une sur l'autre.

3. Emplâtre témoin selon la revendication 1 ou 2, **caractérisé en ce que** la bille (5) résistant à la pression est placée sur la face inférieure (3) de l'emplâtre (1) qui, lors de l'application de ce dernier, est orientée vers la plante du pied.

4. Emplâtre témoin selon au moins une des revendications 1 à 3, **caractérisé en ce que** le diamètre de la bille (5) résistant à la pression est choisi dans un ordre de grandeur compris entre 1,5 et 5 mm, de préférence entre 2 et 4 mm, de telle sorte qu'un patient atteint de pied diabétique ne saura pas faire, à coup sûr, la différence entre un emplâtre avec bille (5) qui, pour l'application, est fixé sous la plante du pied, et un emplâtre sans bille.

5. Emplâtre témoin selon au moins une des revendications 1 à 4, **caractérisé par** un mode de fourniture sous forme d'ensemble constitué d'un emplâtre (1) avec bille (5) et d'un emplâtre sans bille.

6. Emplâtre témoin selon au moins une des revendications 1 à 5, **caractérisé en ce qu'**une feuille témoin de pression (6) incorporée dans l'emplâtre est placée sur la face supérieure (8) de l'emplâtre (1) qui, lors de l'application, est orientée en direction opposée à la plante du pied.
